# EUROPÄISCHE PATENTSCHRIFT

(11) **EP 0 688 771 B1**
(45) Veröffentlichungstag und Bekanntmachung des Hinweises auf die Patenterteilung: **11.07.2001**
(21) Anmeldenummer: 95109191.7
(22) Anmeldetag: 14.06.1995
(51) Int. Cl.: C07D 401/04, C07D 417/04, C07D 421/04, A61K 31/445, A61K 31/425, A61K 31/40

(54) **Verwendung von Lactamverbindungen als pharmazeutische Wirkstoffe**
Use of lactam compounds as pharmaceutical agents
Utilisation de composés lactames comme agents pharmaceutiques

(30) Priorität: 24.06.1994 DE 4422237
(43) Veröffentlichungstag der Anmeldung: 27.12.1995
(73) Patentinhaber: Grünenthal GmbH, 52078 Aachen (DE)
(72) Erfinder: Böhlke, Horst, Dr., D-52222 Stolberg (DE); Finkam, Michael, Dr., D-52066 Aachen (DE); Zimmer, Oswald, Dr., D-52146 Würselen (DE); Schneider, Johannes, Dr., D-52223 Stolberg (DE); Wnendt, Stephan, Dr., D-52076 Aachen (DE); Zwingenberger, Kai, Dr., D-52076 Aachen (DE)

(56) Entgegenhaltungen:
- EP-A- 0 054 672
- WO-A-92/14455
- WO-A-94/20085
- US-A- 3 705 162
- CHEMICAL ABSTRACTS, vol. 113, no. 3, 16. Juli 1990, Columbus, Ohio, US; abstract no. 17466f, & ARZNEIMITTEL FORSCHUNG. DRUG RESEARCH, Bd.40, Nr.1, 1990, AULENDORF DE Seiten 32 - 36 H.P. BUECH ET AL.
- CHEMICAL ABSTRACTS, vol. 112, no. 1, 1. Januar 1990, Columbus, Ohio, US; abstract no. 7314v, & ARCHIV DER PHARMAZIE, Bd.322, Nr.8, 1989, WEINHEIM DE Seiten 499 - 505 J. KNABE ET AL.
- CHEMICAL ABSTRACTS, vol. 107, no. 11, 14. September 1987, Columbus, Ohio, US; abstract no. 96568t, & JUSTUS LIEBIGS ANNALEN DER CHEMIE, Bd.7, 1987, WEINHEIM DE Seiten 647 - 648 G. BLASCHKE ET AL.

## Beschreibung

Die Erfindung betrifft die Verwendung von Lactamverbindungen als pharmazeutische Wirkstoffe, neue Lactamverbindungen sowie Verfahren zu deren Herstellung.

Die überschießende Bildung von Zytokinen wie Tumornekrosefaktor-α (TNF-α), Interleukin-2 (IL-2) und Interferon-γ (IFN-γ), spielt eine zentrale Rolle in der Pathogenese des Graft-versus-host-Syndroms, der Transplantatabstoßung und immunologisch ausgelöster Erkrankungen, beispielsweise Morbus Behcet, aphthöser Stomatitis, Erythema nodosum leprosum, Morbus Boeck, kutanem Lupus erythematodes und rheumatoider Arthritis. Durch Modulation der Zytokinfreisetzung, beispielsweise mit Cyclosporin A oder Glucocorticoiden, können diese pathologischen Situationen behandelt werden. Ein Problem in der Therapie mit Immunsuppressiva besteht jedoch darin, daß durch die Blockierung des Immunsystems häufig opportunistische Infektionen, beispielsweise Mykosen, Pneumonien und Viruserkrankungen auftreten. Daher stellen Wirkstoffe, die eine spezifische zelluläre Immunreaktion dämpfen, aber nicht vollständig unterdrücken, einen Fortschritt für die Therapie dar. Allerdings ist die Behandlung mit den zur Verfügung stehenden Verbindungen nicht immer erfolgreich, so daß ein Bedarf an weiteren immunologisch wirksamen Substanzen besteht.

Aus der Patentanmeldung WO-A-94 20085, welche unter Artikel 54 (3) des Europäischen Patentübereinkommens fällt, ist die Verwendung von Thalidomid und verschiedenen verwandten Verbindungen wie Thalidomidvorläufer, -analoga, -metabolite und -hydrolyseproprodukte zur Reduktion unerwünschter Angiogenese und zur Immunsuppression bekannt. In WO-A-92 14455 ist die Verwendung von Thalidomid und Thalidomidderivaten zur Reduktion erhöhter TNF-α-Konzentrationen beschrieben. Der Wirkstoff Thalidomid wird seit Ende der 60er Jahre erfolgreich bei der Behandlung des Erythema nodosum leprosum eingesetzt und wirkt darüber hinaus bei einer Reihe weiterer Erkrankungen, beispielsweise Morbus Behcet, Lupus erythematodes, Stomatitis aphthosa und dem Graftversus-host-Syndrom (The Lancet 1988, 117; N. Engl. J. Med. 326, 1055 (1992); Eur. J. Dermatol. 4, 9 (1994)). Bei der Behandlung mit Thalidomid kommt es im Gegensatz zu anderen Immunsuppressiva nicht zu einer generellen Einschränkung der Immunkompetenz, so daß Infektionen mit opportunistischen Erregern als Folge der Therapie nicht beobachtet werden. Dieser Befund korreliert mit der Beobachtung, daß Thalidomid im Gegensatz zu typischen Immunsuppressiva die systemische Ausschüttung der Zytokine TNF-α, IL-2 und IFN-γ sowohl in vivo als auch in vitro reduziert, diese jedoch selbst bei hoher Dosierung lokal nicht vollständig unterbindet. Anders als bei typischen Immunsuppressiva, beispielsweise Cyclosporin A und Dexamethason, wird auch die Proliferation immunkompetenter Zellen durch Thalidomid nicht unterbunden.

Es wurde nunmehr gefunden, daß bestimmte Lactamverbindungen in pharmakologischen Modellen gleich gut oder besser als Thalidomid wirken, jedoch im Gegensatz zu Thalidomid in diesen Modellen nicht teratogen sind.

Gegenstand der Erfindung ist daher eine Lactam-Verbindung der Formel I 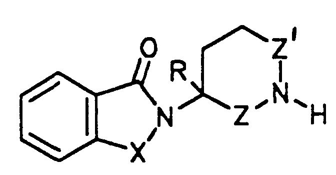 in der X S oder Se und R eine C₁₋₆-Alkyl- oder die Benzylgruppe bedeutet, Z und Z' verschieden sind und CH₂ oder CO bedeuten oder Z und Z' gleich sind und CO bedeuten, in racemischer oder optisch aktiver Form zur Verwendung als pharmazeutischer Wirkstoff.

Die Lactamverbindungen der Formel I und insbesondere solche Lactamverbindungen der Formel I, in der R eine C₁₋₃-Alkylgruppe, das heißt eine Methyl-, Ethyl-, Propyl- oder Isopropylgruppe bedeutet, besitzen ein breites immunomodulatorisches, insbesondere immunosuppressives Wirkungsspektrum, so daß die Lactamverbindungen der Formel I vorzugsweise zur Verwendung als Immunmodulatoren und/oder Immunsuppressiva geeignet sind.

Darüber hinaus eignen sich Lactamverbindungen der Formel I insbesondere auch zur Reduktion der Neoangiogenese. Neoangiogenese, d. h. pathologische Gefäßneubildung, ist eine unerwünschte Folge immunologisch ausgelöster Erkrankungen wie Morbus Crohn und Infektionen mit Mycobakterium leprae (Int. J. Leprosy 9, 193 (1941)), aber auch granulamatöser Erkrankungen.

Zur Behandlung von Erkrankungen des Immunsystems, beispielsweise der Graft-versus-host-disease, der Transplant-Rejektion, der Behcet-Krankheit, von Lupus erythematodes, von Autoimmunerkrankungen als Spätfolge von Infektionen, von Morbus Crohn oder der Kawasaki-Krankheit können Lactamverbindungen der Formel I oral, intravenös, intraperitoneal, intradermal, intramuskulär und intranasal sowie örtlich, zum Beispiel auf Infektionen an der Haut, der Schleimhäute und an den Augen, appliziert werden. Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 1 bis 10 mg/kg einer Lactamverbindung der Formel I appliziert.

Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Lactamverbindungen der Formel I in einem Depot in gelöster Form oder in einem Pflaster gegebenenfalls unter Zusatz von die Hautpenetration fördernden Mitteln sind geeignete perkutane Applikationszubereitungen. Oral oder perkutan anwendbare Zubereitungsformen können die Lactamverbindung der Formel I verzögert freisetzen.

Alle vorstehend aufgeführten pharmazeutischen Zubereitungsformen sind an sich bekannt, und da die erfindungsgemäß zu verwendenden Verbindungen chemisch stabil sind, wirft ihre Einarbeitung in diese Zubereitungsformen für den Fachmann keinerlei Probleme auf. Bei der Herstellung der Arzneimittel muß mit der üblichen Sorgfalt bei Auswahl der Hilfsstoffe, beispielsweise Trägermaterialien, Füllstoffe, Lösungsmittel, Verdünnungsmittel, Farbstoffe, Geschmackskorrigenzien, Bindemittel und Tablettensprengstoffe vorgegangen werden, und insbesondere bei der Herstellung von parenteral anzuwendenden Zubereitungsformen ist auf Stabilität und, sofern diese in flüssiger Form vorliegen, Isotonie zu achten.

Weiterer Gegenstand der Erfindung sind die bisher nicht bekannten Lactamverbindungen der Formel I 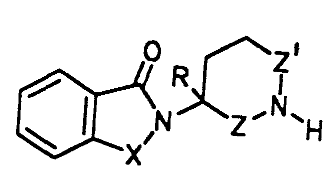 in der X, S oder Se und R eine C₁₋₆_Alkyl- oder die Benzylgruppe bedeutet, Z und Z' verschieden sind und CH₂ oder CO bedeuten oder Z und Z' gleich sind und CO bedeuten mit der Maßgabe, daß X kein S bedeutet, wenn Z CO und Z' CH₂ ist, in racemischer oder optisch aktiver Form.

Die erfindungsgemäßen Verbindungen der Formel I, von denen diejenigen Verbindungen, die als Rest R eine Methyl-, Ethyl-, Propyl- oder Isopropylgruppe besitzen, bevorzugt werden, sind konfigurationsstabil.

Ferner ist Erfindungsgegenstand ein Verfahren zur Herstellung einer Verbindung der Formel I 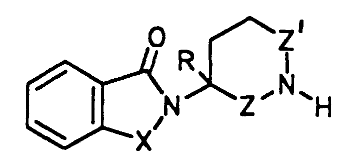 in der X S oder Se und R eine C₁₋₆-Alkyl- oder die Benzylgruppe bedeutet, Z und Z' verschieden sind und CH₂ oder CO bedeuten oder Z und Z' gleich sind und CO bedeuten, mit der Maßgabe, daß X kein S bedeutet, wenn Z CO und Z' CH₂ ist, wobei dieses Verfahren dadurch gekennzeichnet ist, daß eine Verbindung der Formel IV 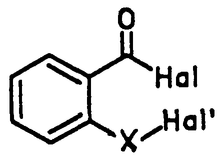 in der Hal und Hal' gleich oder verschieden sind und Chlor oder Brom bedeuten, mit einer Verbindung der Formel V 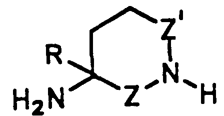 in Gegenwart einer Base bei Temperaturen zwischen -20° C und +50° C umgesetzt wird.

Vorzugsweise wird eine Verbindung der Formel IV, in der Hal und Hal' Chlor bedeuten, in Gegenwart eines Lösungsmittels, beispielsweise Dimethylformamid, und einer Base, beispielsweise Triethylamin, Pyridin und/oder Diisopropylethylamin, umgesetzt. Verbindungen der Formel IV lassen sich nach den in EP 54 672 und EP 354 412 beschriebenen Verfahren herstellen.

Verbindungen der Formel VI 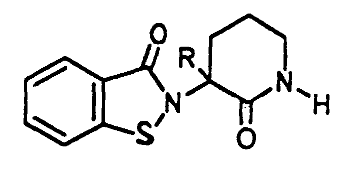 sind nach dem in EP 54 672 beschriebenen Verfahren zugänglich.

### Beispiele

Die für Lösungsmittelgemische angegebenen Verhältnisse beziehen sich auf das Volumen.

### Beispiel 1

### 2-(3-Methyl-2-oxo-piperidin-3-yl)-benzo[d]iso-selenazol-3-on

Zu einer Lösung von 0,47 g 3-Amino-3-methyl-piperidin-2-on in 10 ml Dimethylformamid wurden bei -20° C unter Rühren nacheinander eine Lösung von 0,93 g o-Chlorselenobenzoesäurechlorid in 5 ml Dimethylformamid und 1 ml Triethylamin getropft. Es wurde 22 Stunden bei dieser Temperatur gerührt und dann tropfenweise mit 5 ml Wasser versetzt. Die ausgefallenen Kristalle wurden durch Absaugen isoliert und aus 20 ml Ethanol/Essigsäureethylester (3/5) umkristallisiert. Dabei fielen 0,49 g (43,4 %) 2-(3-Methyl-2-oxo-piperidin-3-yl)-benzo[d]isoselenazol-3-on in Form weißer Kristalle an, die zwischen 263° C und 270° C schmolzen.
- ¹H-NMR (DMSO-d₆):: 1,72 (s, 3H, CH₃); 1,70 - 1,90 (m, 3H, CH₂, CH); 2,44 - 2,55 (m, 1H, CH); 3,17 - 3,34 (m, 1H, CH₂); 7,38 - 8,04 (m, 5H, CHₐᵣ, NH) ppm.

### Beispiel 2

### 2-(3-Methyl-2-oxo-piperidin-3-yl)-benzo[d]isothiazol-3-on

Die Lactamverbindung wurde durch Umsetzung von 3-Amino-3-methyl-piperidin-2-on mit o-Chlormercaptobenzoesäurechlorid unter den in Beispiel 10 der EP 54 672 angegebenen Bedingungen hergestellt.

### Pharmakologische Untersuchungen

### Wirksamkeit erfindungsgemäßer Verbindungen im Tiermodell

Für die in-vivo Charakterisierung der immunpharmakologischen Wirkungen der Lactam-Verbindungen der Formel I wurde ein Testmodell gewählt, in dem T-Lymphozyten stimuliert werden. Die Relevanz des immunpharmakologischen Testmodells, dessen Zielzelle unter anderen die T-Zelle ist, ergibt sich aus der zentralen Bedeutung der T-Zelle in verschiedenen immunologischen Krankheiten. Dazu gehören: Die Graft-versus-host-Disease (GvHD), die Transplantat-Rejektion (Host-versus-Graft), Autoimmunerkrankungen als Spätfolge von Infektionen (z. B. rheumatisches Fieber, rheumatoide Arthritis), die Behcet Krankheit (mukokutane Ulzerationen) und die Kawasaki Krankheit (multisytemische Vaskulitiden).

Die Stimulation von T-Zellen wurde durch i.v.-Applikation des Staphylokokken Enterotoxin B (SEB; 200 µg) an Galaktosaminvorbehandelten Balb/c-Mäusen erreicht. SEB gehört zur Gruppe der Superantigene, die in Verbindung mit dem MHC-II-Komplex (Major Histocampatibility Complex, Klasse II) einer Antigen-präsentierenden Zelle eine Aktivierung des T-Zell-Rezeptors (TZR) bewirken. Als Parameter für die T-Zell-Aktivierung wurden die Serumkonzentrationen des Zytokins IL-2 bestimmt. IL-2 wird von T_{H1}-Zellen gebildet. IL-2 wurde im Serum mittels eines kommerziellen ELISA-Tests, der spezifisch murines IL-2 detektiert, bestimmt. Die Injektion von SEB führte zu einem zeitabhängigen Anstieg der Serum-IL-2-Spiegel mit einem deutlichen Maximum bei 2 Stunden nach SEB-Applikation. Die Herkunft des im Serum gemessenen IL-2 aus T-Zellen konnte dadurch verifiziert werden, daß T-Zell-defiziente SCID-Mäuse im Gegensatz zu Balb/c-Mäusen nach Injektion der gleichen Menge SEB kein IL-2 bildeten.

Die erfindungsgemäß zu verwendenden Verbindungen wurden in einer wäßrigen 1 %igen Carboxymethylcelluloselösung (1 % CMC) gelöst und in Dosierungen von 10 mg/kg und im Volumen von 1 ml/kg 30 Minuten vor der SEB-Injektion intraperitoneal (i. p.) appliziert. Tiere der Kontrollgruppe erhielten zum gleichen Zeitpunkt 1 ml/kg Vehikellösung (1 % CMC) i. p. appliziert. Die Serum-IL-2-Konzentrationen wurden 2 Stunden nach SEB-Applikation (d. h. 2,5 Stunden nach Substanzapplikation) bestimmt. In Tabelle 1 sind die maximalen Hemmeffekte (in %) der Serum-IL-2-Spiegel in den mit einer Lactam-Verbindung behandelten Gruppen im Vergleich zur Kontrollgruppe dargestellt. Die Prozent-Angaben stellen Mittelwerte mit mittlerem Fehler der Mittelwerte von jeweils n = 6 - 8 Einzelversuchen dar. Über eine Regressionsgerade wurden die Dosierungen errechnet, die zu einer 40 %-igen Reduktion der Serum-IL-2-Konzentrationen führten (ED40-Werte).

**Tabelle 1**

| Wirkung von Lactam-Verbindungen der Formel I auf die IL-2-Freisetzung in vivo | | |
|---|---|---|
| Lactam-Verbindung hergestellt gemäß Beispiel | Maximale Hemmung des Serum-IL-2-Anstieges in % (Dosis in Klammern) | ED40 [mg/kg i.p.] |
| 1 | 43 ± 5 ( 10 mg/kg) | ca. 20 |
| 2 | 52 ± 3 ( 10 mg/kg) | <10 |
| Thalidomid (Vergleich) | 56 ± 10 (400 mg/kg) | 171 |

Vergleichende Untersuchungen zeigten, daß die erfindungsgemäß zu verwendenden Lactam-Verbindungen im Gegensatz zu Glucokorticoiden die T-Zellaktivität auch dann hemmten, wenn sie 30 Minuten vor der T-Zellstimulation durch SEB verabreicht wurden. Für eine hemmende Wirkung durch Glukokortikoide war es dagegen erforderlich, daß diese Substanzen 18 Stunden vor der Stimulation der T-Zellen appliziert wurden.

### Wirksamkeit erfindungsgemäßer Verbindungen auf humane Zellen in vitro

Die Freisetzung von Zytokinen kann in vitro an humanen mononukleären Zellen des peripheren Blutes, also T-Zellen, B-Zellen und Monozyten nach Stimulation mit Lipopolysaccharid (LPS) oder dem Toxic Shock Syndrome Toxin-1 (TSST-1) untersucht werden. LPS ist ein Bestandteil der bakteriellen Zellwand und stimuliert Monozyten und Makrophagen. Bei TSST-1 handelt es sich um ein bakterielles Superantigen, das sowohl T-Zellen als auch Monozyten/Makrophagen aktiviert. Superantigene binden an die Vß-Kette des T-Zellrezeptors und an den MHC Klasse II und simulieren dadurch die Erkennung eines vom MHC Klasse II präsentierten Antigen durch einen spezifischen T-Zellrezeptor. Die Aktivierung der Zellen durch LPS oder TSST-1 führt zur Freisetzung von TNF-α, IFN-γ und anderen Zytokinen.

Mononukleäre Zellen wurden aus dem heparinisierten Blut von mindestens drei freiwilligen Spendern gewonnen. Hierzu wurden je 20 ml Blut nach bekannten Methoden über einen Ficoll-Paque-Gradienten aufgetrennt, die Zellen geerntet und dreimal mit einem Zellkulturmedium gewaschen. Dieses Zellkulturmedium bestand aus RPMI 1640 Medium supplementiert mit 2 mM Glutamin (Life Technologies, Eggenstein, Deutschland), 10 % fötalem Kälberserum (Life Technologies), 50 µg/ml Streptomycin (Sigma, Deisenhofen, Deutschland), 50 IU/ml Penicillin (Sigma) und 100 µM b-Mercaptoethanol (Merck, Darmstadt, Deutschland). Die mononukleären Zellen wurden in 15 ml Zellkulturmedium aufgenommen und in 1 ml-Ansätzen in sterilen 24-Loch-Inkubationsplatten (Sigma) aufgeteilt. Den 1-ml-Ansätzen wurde jeweils 1 µl Dimethylsulfoxid (DMSO) oder 1 µl einer DMSO-Lösung, enthaltend 5 Gew.-% einer erfindungsgemäß zu verwendenden Verbindung, zugesetzt. Nach einstündiger Inkubation im CO₂-Brutschrank (5 % CO₂, 90 % Luftfeuchtigkeit) wurden den Ansätzen mit einer erfindungsgemäß zu verwendenden Verbindung jeweils 2,5 µg LPS (von E. coli 0127: B8, Sigma) oder 0,1 µg TSST-1 (Sigma) zugefügt. Die Inkubation der Kulturen wurde 20 Stunden fortgesetzt. Die Konzentration von TNF-α und IFN-γ in den Zellkulturüberständen wurde mit kommerziellen ELISA-Tests (Boehringer-Mannheim, Deutschland; Endogen, Boston, USA) bestimmt.

In Tabelle 2 ist der Einfluß von erfindungsgemäß zu verwendenden Lactam-Verbindungen sowie von Thalidomid auf die LPS-induzierte Freisetzung von TNF-α dargestellt. Erfindungsgemäße Lactam-Verbindungen hemmten die TNF-α-Freisetzung stärker als Thalidomid.

**Tabelle 2:**

| Hemmung der TNF-α-Freisetzung von Lactam-Verbindungen der Formel I | |
|---|---|
| Lactam-Verbindung hergestellt gemäß Beispiel | Hemmung der TNF-α Freisetzung in (%) bei einer Konzentration von 50 µg/ml |
| 1 | 93 ± 1 |
| 2 | 93 ± 9 |
| Thalidomid (Vergleich) | 54 ± 7 |

Die Resultate aus den in vivo und in vitro Untersuchungen zeigen, daß die erfindungsgemäß zu verwendenden Verbindungen in der Lage sind, die Aktivierung immunkompetenter Zellen zu hemmen. Als Maß für die Aktivierung der Zellen diente die Freisetzung der Zytokine IL-2, TNF-α und IFN-γ. Die dämpfende Wirkung der erfindungsgemäß zu verwendenden Verbindungen auf die Ausschüttung dieser aktivierungsspezifischen Zytokine weist darauf hin, daß diese Verbindungen für die Therapie von Krankheiten geeignet sind, die mit einer Überaktivität immunkompetenter Zellen einhergehen. Dabei können diese Verbindungen sowohl prophylaktisch als auch kurativ eingesetzt werden.

## Patentansprüche

1. Lactam-Verbindung der Formel I 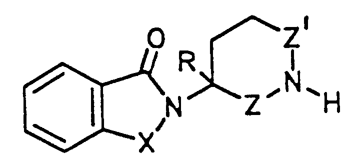 in der X S oder Se und R eine C₁₋₆-Alkyl- oder die Benzylgruppe bedeutet, Z und Z' verschieden sind und CH₂ oder CO bedeuten oder Z und Z' gleich sind und CO bedeuten,
in racemischer oder optisch aktiver Form zur Verwendung als pharmazeutischer Wirkstoff.

2. Lactam-Verbindung gemäß Anspruch 1, in der R eine C₁₋₃-Alkylgruppe bedeutet, zur Verwendung gemäß Anspruch 1.

3. Lactam-Verbindung gemäß Anspruch 1 und/oder Anspruch 2 zur Verwendung als Immunmodulator und/oder Immunsuppressivum.

4. Lactam-Verbindung gemäß Anspruch 1 und/oder Anspruch 2 zur Reduktion unerwünschter Neoangiogenese.

5. Lactam-Verbindungen der Formel I 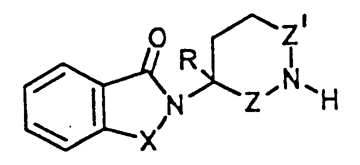 in der X S oder Se und R eine C₁₋₆-Alkyl- oder die Benzylgruppe bedeutet, Z und Z' verschieden sind und CH₂ oder CO oder Z und Z' gleich sind und CO bedeuten, mit der Maßgabe, daß X kein S bedeutet, wenn Z CO und Z' CH₂ ist, in racemischer oder optisch aktiver Form.

6. Lactam-Verbindungen nach Anspruch 5, dadurch gekennzeichnet, daß R eine C₁₋₃-Alkylgruppe ist.

7. Verfahren zur Herstellung einer Lactam-Verbindung der Formel I gemäß Anspruch 5, dadurch gekennzeichnet, daß eine Verbindung der Formel IV 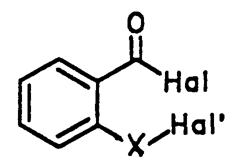 in der Hal und Hal' gleich oder verschieden sind und Chlor oder Brom bedeuten, mit einer Verbindung der Formel V 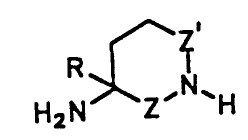 in Gegenwart einer Base bei Temperaturen zwischen -20° und +50° C umgesetzt wird.

## Claims

1. Lactam compound of the formula I 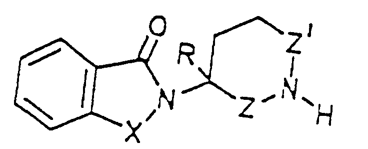 in which X denotes S or Se and R denotes a C₁₋₆-alkyl group or the benzyl group, Z and Z' are different and denote CH₂ or CO, or Z and Z' are the same and denote CO, in the racemic or optically active form, for use as a pharmaceutical active substance.

2. Lactam compound according to claim 1, in which R denotes a C₁₋₃-alkyl group, for use according to claim 1.

3. Lactam compound according to claim 1 and/or claim 2, for use as an immunomodulator and/or immunosuppressive agent.

4. Lactam compound claim 1 and/or claim 2 for reducing undesirable neoangiogenesis.

5. Lactam compounds of the formula I 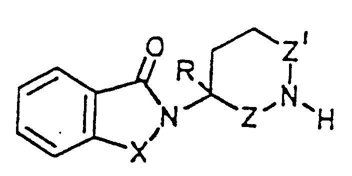 in which X denotes S or Se and R denotes a C₁₋₆-alkyl group or the benzyl group, Z and Z' are different and denote CH₂ or CO, or Z and Z' are the same and denote CO, with the proviso that X does not denote S when Z is CO and Z' is CH₂, in the racemic or optically active form.

6. Lactam compounds according to claim 5, characterised in that R is a C₁₋₃-alkyl group.

7. Process for the preparation of a lactam compound of the formula I according to claim 5, characterised in that a compound of the formula IV 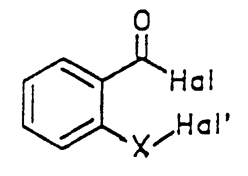 in which Hal and Hal' are the same or different and denote chlorine or bromine, is reacted with a compound of the formula V 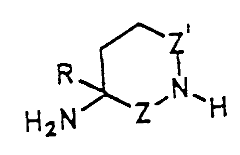 in the presence of a base at temperatures between -20°C and +50°C.

## Revendications

1. Composé du type d'un lactame, de formule I : 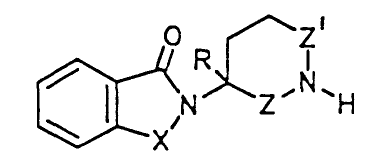 dans laquelle X représente S ou Se, et R est un groupe alkyle en C₁ à C₆ ou le groupe benzyle, Z et Z' sont différents et représentent CH₂ ou CO, ou bien Z et Z' sont identiques et représentent CO,
sous une forme racémique ou optiquement active, destiné à être utilisé comme substance pharmaceutique active.

2. Composé du type d'un lactame suivant la revendication 1, dans lequel R représente un groupe alkyle en C₁ à C₃, destiné à être utilisé conformément à la revendication 1.

3. Composé du type d'un lactame suivant la revendication 1 et/ou la revendication 2, destiné à être utilisé comme immunomodulateur et/ou comme immunosuppresseur.

4. Composé du type d'un lactame suivant la revendication 1 et/ou la revendication 2, destiné à être utilisé pour réduire une néoangiogénèse indésirable.

5. Composés du type de lactames de formule I : 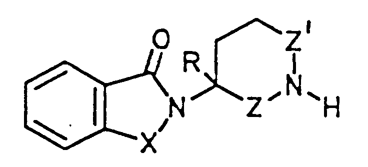 dans laquelle X représente S ou Se, et R représente un groupe alkyle en C₁ à C₆ ou le groupe benzyle, Z et Z' sont différents et représentent CH₂ ou CO, ou bien Z et Z' sont identiques et représentent CO,
sous réserve que X ne représente pas S lorsque Z est un groupe CO et Z' est un groupe CH₂, sous une forme racémique ou optiquement active.

6. Composés du type de lactames suivant la revendication 5, caractérisés en ce que R est un groupe alkyle en C₁ à C₃.

7. Procédé de production d'un composé du type d'un lactame de formule I suivant la revendication 5, caractérisé en ce que l'on fait réagir un composé de formule IV : 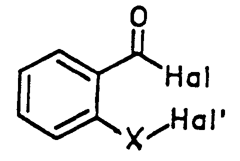 dans laquelle Hal et Hal' sont égaux ou différents et représentent le chlore ou le brome, avec un composé de formule V : 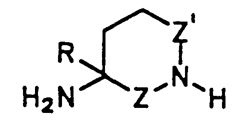 en présence d'une base, à des températures comprises entre -20°C et +50°C.
